# EUROPEAN PATENT SPECIFICATION

(11) **EP 1 326 850 B1**
(45) Date of publication and mention of the grant of the patent: **16.11.2005**
(21) Application number: 01987749.7
(22) Date of filing: 09.10.2001
(51) Int. Cl.: C07D 263/62, C07D 277/66, C07D 235/20, C07D 417/14, C07D 405/14, C07D 413/14

(54) **A PROCESS FOR THE PREPARATION OF BIS-BENZAZOLYL COMPOUNDS**
VERFAHREN ZUR HERSTELLUNG VON BIS-BENZAZOLYL VERBINDUNGEN
PROCEDE DE PREPARATION DE COMPOSES BIS-BENZAZOLYLE

(30) Priority: 18.10.2000 EP 00810961
(43) Date of publication of application: 16.07.2003
(73) Proprietor: Ciba Specialty Chemicals Holding Inc., 4057 Basel (CH)
(72) Inventor: ELIU, Victor, Paul, 79539 Lörrach (DE); HAUSER, Julia, 79639 Grenzach-Wyhlen (DE)
(86) International application number: PCT/EP2001/011644
(87) International publication number: WO 2002/032886

(56) References cited:
- EP-A- 0 719 774
- CH-A- 439 292
- GB-A- 1 201 287
- US-A- 2 842 545
- US-A- 3 407 196

## Description

The present invention relates to a process for the preparation of bis-benzazolyl compounds which are useful as optical whitening agents for natural and synthetic materials.

Various methods for the preparation of such compounds are known.

Thus, for example, US Patent 4,508,903 describes the preparation of 4,4'-bis.benzoxazol-, benzthiazol- and benzimidazol-2-ylstilbenes by dimerisation of the corresponding p-chloromethylphenylbenzazoles. However, such methods suffer from the disadvantage that the preparation of the intermediates involves several reaction steps, subsequently rendering poor overall yields.

EP-A-0 719 774 discloses a process of the preparation dibenzoxazolyl thiophenes which comprises reacting a thiophene-2,5-dicarboxylic acid ester with ortho-aminophenols in the presence of a acid catalyst.

U.S. 3,407,196 discloses the preparation of new benzoxazolylstilbenes by reaction of a dicarboxylic acid chloride with ortho-aminophenols in the presence of a solvent such as dichlorobenzene, phthalic acid dibutyl ester or xylene.

CH 439 292 discloses a process for the preparation of thiophene derivatives which comprises reacting a dicarboxylic acid with ortho-aminophenols in the melt.

U.S. 2,842,545 discloses a process for the preparation of α,β-di[aryloxazolyl-(2)]-ethylene compounds which comprises reacting a dicarboxylic acid of functional derivative thereof with an ortho-aminophenol in the presence of a solvent such as xylene.

Of particular practical interest are processes in which dicarboxylic acids or their derivatives are reacted with bifunctional aromatic compounds to form the heterocyclic rings in a single reaction step.

Thus, for example, European Patent 31,296 discloses a process for the preparation of benzoxazolyl and benzimidazolyl compounds by condensation of organic carboxylic acids with o-aminophenols and o-phenylenediamines in a solvent mixture consisting of diphenyl ether and diphenyl in the presence of acidic catalysts. Furthermore, British Patent 1,201,287 describes the preparation of 2,5-bisbenzoxazol-2-yl thiophenes by condensation of thiophene-2,5-dicarboxylic acid with o-aminophenols in, for example, refluxing 1,2,4-trichlorobenzene in the presence of boric acid. Such processes are disadvantageous since they demand extremely high reaction temperatures, resulting in the formation of impurities which are difficult to remove from the final products and, as a consequence, loss of product yields. Furthermore, such high-boiling solvents are also difficult to remove from the reaction products and may further result in crust-formation inside reaction vessels, thus impeding work-up of the final products. Additionally, employment of chlorinated aromatic solvents in the present day is undesirable for ecological reasons.

Surprisingly, a new, advantageous process for the preparation of bis-benzazolyl compounds has now been found, which provides these compounds in high yields of excellent purity under reaction conditions well suited to commercial processes.

Accordingly, the current invention provides a process for the preparation of a compound of the formula 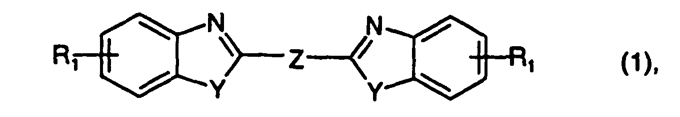 wherein
Y represents -O-, -S- or -N(R₂)-,
R₂ being hydrogen, C₁-C₁₀alkyl or aralkyl;
Z represents a 2,5-furanyl, 2,5-thiophenyl, 4,4'-stilbenyt or a 1,2-ethylenyl residue and
R₁ represents hydrogen, halogen, C₁-C₁₀alkyl, C₁-C₁₀alkoxy, cyano, COOM or SO₃M, M being hydrogen or an alkaline or alkaline earth metal, characterized by reacting a compound of the formula 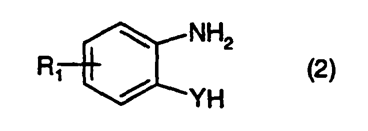 with a dicarboxylic acid of the formula

   HOOC-Z-COOH (3)

   or an ester thereof, Y, Z and R₁ being as previously defined, in N-methylpyrrolidone or N,N-dimethylacetamide, in the presence of an acidic catalyst and, optionally, in the presence of a secondary solvent capable of removing water from the reaction mixture.

The molar ratios of the compound of formula (2) to the compound of formula (3) may vary over wide ranges. However, it is advantageous to react at least two moles of the compound of formula (2) with one mole of the dicarboxylic acid of formula (3). Altematively, a mono- or diester, preferably a diester, of the compound of formula (3) may be employed. Appropriate esters are those derived from a C₁-C₁₀-, preferably C₁-C₄alcohol, diethyl esters being most preferred.

The process of the invention is particularly suitable for the preparation of a compound of formula (1) in which
Y represents -O-, -S- or -N(R₂)-,
R₂ being hydrogen or C₁-C₄alkyl;
Z is as defined previously and
R₁ represents hydrogen or C₁-C₄alkyl and, more especially for compounds of formula (1) in which
Z represents a 2,5-furanyl or a 2,5-thiophenyl residue and also for those in which
Z represents a 4,4'-stilbenyl or a 1,2-ethylenyl residue.

As reaction medium for the process of the invention N-methylpyrrolidone or N,N-dimethylacetamide or mixtures thereof are most preferred. It is also possible to use N-methylpyrrolidone or N,N-dimethylacetamide or mixtures thereof together with a further high boiling inert solvent, e. g. toluene or xylene. The use of N-methylpyrrolidone is especially preferred.

The acidic catalyst employed in the process of the invention may be selected from the group consisting of boric acid, phosphoric acid, titanium C₁-C₄orthoesters or tin derivatives, boric acid or a titanium C₁-C₄orthoester, especially tetrapropyl or tetrabutyl ester, being of preference. The amount of catalyst employed may vary over wide ranges and is dependent on the chemical entity. Thus, for example, amounts varying from 0.01 to 50mote%, based on the amount of compound (2), preferably 0.1 to 30mole% may be used.

Reaction of compounds of the formulae (2) and (3) may be carried out within a wide temperature range, but is preferably within the range of between 100 and 250°C, in particular within a temperature range of between 150 and 200°C.

The presence of a secondary solvent is of particular importance when the compound of formula (3) is in the form of a monoester or, especially, the free dicarboxylic acid. In these cases, water formed during the course of the reaction may be continuously removed from the reaction mixture. Examples of suitable solvents, without the choice being limited thereto, are selected from the group consisting of toluene, the xylenes and isomeric mixtures thereof and pyridine, toluene and xylene being especially effective.

The reaction of the invention is normally carried out under atmospheric pressure. However, under certain circumstances, it may prove advantageous to perform the reaction under higher or lower pressures.

Within the scope of the compounds of formulae (1) and (2), when R₁ represents halogen this may be fluorine, bromine , iodine or, especially, chlorine.

C₁-C₁₀alkyl groups R₁ and/or R₂ may be branched or unbranched such as methyl, ethyl, n-propyl, isopropyl, n-butyl, sec-butyl, isobutyl, t-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, n-nonyl or n-decyl. C₁-C₁₀alkyl esters of compound of formula (3) are substituted correspondingly.

C₁-C₁₀alkoxy groups R₁ may be branched or unbranched such as methoxy, ethoxy, n-propoxy, isopropoxy, n-butoxy, sec-butoxy, isobutoxy, t-butoxy, 2-ethylbutoxy, n-pentoxy, isopentoxy, 1-methylpentoxy, 1,3-dimethylbutoxy, n-hexoxy, 1-methylhexoxy, n-heptoxy, isoheptoxy, 1,1,3,3-tetramethylbutoxy, 1-methylheptoxy, 3-methylheptoxy, n-octoxy, 2-ethylhexoxy, 1,1,3-trimethylhexoxy, 1,1,3,3-tetramethylpentoxy, n-nonoxy or n-decoxy.

Aralkyl groups R₂ may be benzyl or phenethyl which may be substituted by halogen, C₁-C₁₀alkyl or C₁-C₁₀alkoxy groups or, preferably, unsubstituted.

The alkaline or alkaline earth metal M may be selected from the group consisting of K, Na, Ca or Mg, but is preferably K or Na.

The following Examples further illustrate the present invention, without intending to be restrictive thereto:

### Example 1

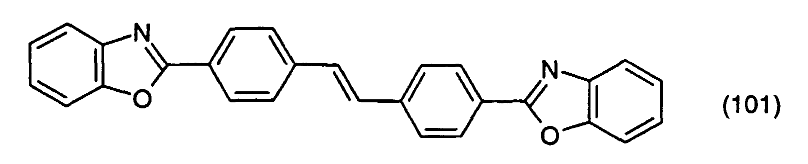

250g of N-methylpyrrolidone are charged to a reaction vessel and 82g of 98% stilbene-4,4'-dicarboxylic acid, followed by 75g of 99% 2-aminophenol, 10g of boric acid and 30g of xylene are added with stirring. The apparatus, equipped with a Dean and Stark water trap, is evacuated and the vacuum released with nitrogen. The light yellow suspension is heated to 195°C and stirred at this temperature for 18 hours, during which time 23-25ml of water and approximately 25g of xylene are distilled off through the water trap. The reaction mixture is cooled to 20°C and stirring continued for 1 hour at this temperature. The yellow suspension is filtered, washed with 100g of N-methylpyrrolidone to give 350g of a brown solution which may be used as solvent for a further charge and then with three 80g portions of water. The resulting press-cake is dried under a vacuum of 50mbar at 100°C to yield 120g of the compound of formula (101) as a yellow solid, characterized by a UV absorption maximum λₘₐₓ at 368nm with an extinction coefficient ε of 71000.

### Example 2

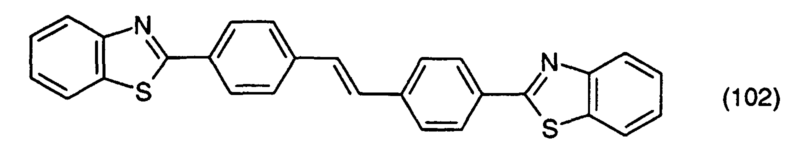

By following the procedure described in Example 1, but replacing the 2-aminophenol by 82g of 98% 2-thiophenol and the boric acid by 3g of titanic acid tetra-isopropyl ester, there are obtained 115g of the compound of formula (102) as a yellow solid, characterized by a UV absorption maximum λₘₐₓ at 375nm with an extinction coefficient ε of 62000 and by the following ¹H-NMR data in D₆-DMSO:
8.12, 4H, m; 8.00, 6H, m; 7.85, 4H, m and 7.48, 4H, m.

### Example 3

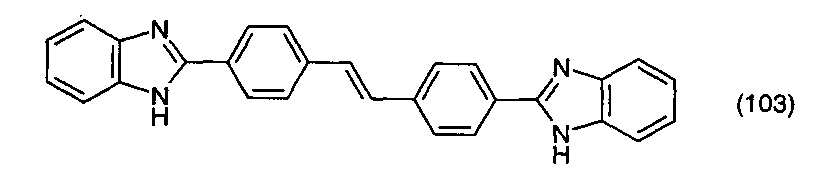

By following the procedure described in Example 1, but replacing the 2-aminophenol by 72g of 99% 1,2-phenylenediamine, there are obtained 110g of the compound of formula (103) as a yellow solid, characterized by a UV absorption maximum λₘₐₓ at 370nm with an extinction coefficient ε of 63000 and by the following ¹H-NMR data in D₆-DMSO:
13.0, 2H, s; 8.22, 4H, d, j= 7Hz; 7.80, 4H, d, j=7Hz; 7.68, 2H, d, j=7Hz; 7.54, 2H, d, j=7Hz; 7.48, 2H, s and 7.22, 4H, t, j=7Hz.

### Example 4

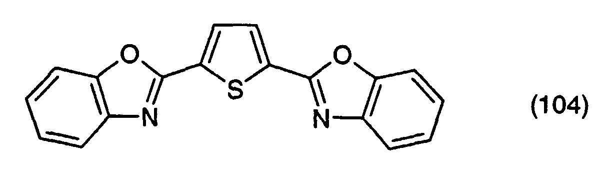

200g of N-methylpyrrolidone are charged to a reaction vessel and 52g of 98% thiophene-2,5-dicarboxylic acid, followed by 72g of 99% 2-aminophenol, 10g of boric acid and 30g of toluene are added with stirring. The apparatus, equipped with a Dean and Stark water trap, is evacuated and the vacuum released with nitrogen. The light yellow suspension is heated to 185°C and stirred at this temperature for 12 hours, during which time 23-25ml of water and approximately 25g of toluene are distilled off through the water trap. The reaction mixture is cooled to 20°C and stirring continued for 1 hour at this temperature. The yellow suspension is filtered, washed with 100g of N-methylpyrrolidone to give 300g of a brown solution which may be used as solvent for a further charge and then with three 80g portions of water. The resulting press-cake is dried under a vacuum of 50mbar at 100°C to yield 75g of the compound of formula (104) as a yellow solid, characterized by a UV absorption maximum λₘₐₓ at 372nm with an extinction coefficient ε of 52000 and by the following
¹H-NMR data in D₆-DMSO:
8.10, 2H, s; 7.82, 4H, m and 7.50, 4H, m.

### Example 5

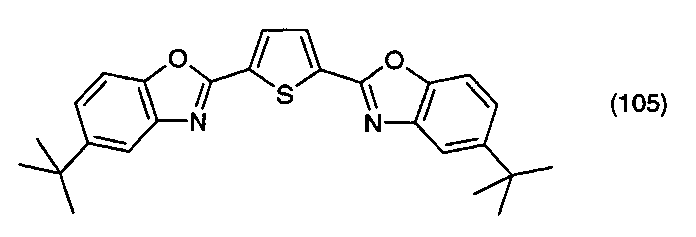

By following the procedure described in Example 4, but replacing the 2-aminophenol by 110g of 2-amino-4-t-butylphenol, the boric acid by 2.2g of isopropyl-ortho-titanate and the toluene by 30g of xylene, there are obtained 125g of the compound of formula (105) as a yellow solid, characterized by a UV absorption maximum λₘₐₓ at 375nm with an extinction coefficient ε of 51000 and by a singlet at 1.30ppm in the ¹H-NMR spectrum in D₆-DMSO.

### Example 6

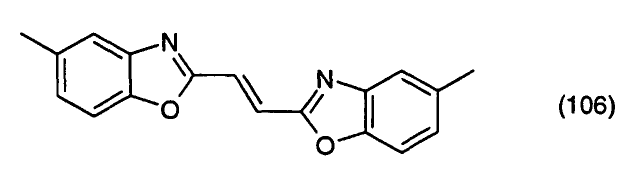

200g of N,N-dimethylacetamide are charged to a reaction vessel and 35g of 98% fumaric acid, followed by 82g of 2amino-4-methylphenol, 10g of boric acid and 30g of xylene are added with stirring. The apparatus, equipped with a Dean and Stark water trap, is evacuated and the vacuum released with nitrogen. The light yellow suspension is heated to 160°C and stirred at this temperature for 10 hours, during which time 23-25ml of water and approximately 25g of xylene are distilled off through the water trap. The reaction mixture is cooled to 20°C and stirring continued for 1 hour at this temperature. The yellow suspension is filtered, washed with 100g of N,N-dimethylacetamide and then with three 80g portions of water. The resulting press-cake is dried under a vacuum of 50mbar at 100°C to yield 85g of the compound of formula (106) as a yellow solid, characterized by a UV absorption maximum λₘₐₓ at 365nm with an extinction coefficient ε of 42000.

### Example 7

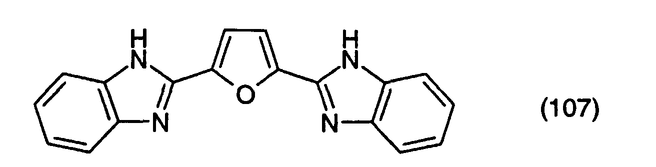

200g of N-methylpyrrolidone are charged to a reaction vessel and 65g of furan-2,5-dicarboxylic acid, followed by 72g of 99% 1,2-phenylenediamine and 10g of boric acid are added with stirring. The apparatus, equipped with a Dean and Stark water trap, is evacuated and the vacuum released with nitrogen. The light yellow suspension is heated to 175°C and stirred at this temperature for 12 hours, during which time 28g of ethanol are distilled off under a weak vacuum. The resulting solution is cooled to 20°C and stirring continued for 1 hour at this temperature. The yellow suspension is filtered, washed with 100g of N-methylpyrrolidone to give 300g of a brown solution which may be used as solvent for a further charge and then with three 80g portions of water. The resulting press-cake is dried under a vacuum of 50mbar at 100°C to yield 95g of the compound of formula (107) as a yellow solid, characterized by a UV absorption maximum λₘₐₓ at 375nm with an extinction coefficient ε of 42000.

## Claims

1. A process for the preparation of a compound of the formula 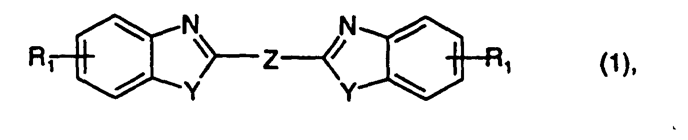 wherein
Y represents -O-, -S- or -N(R₂)-,
R₂ being hydrogen, C₁-C₁₀alkyl or aralkyl;
Z represents a 2,5-furanyl, 2,5-thiophenyl, 4,4'-stilbenyl or a 1,2-ethylenyl residue and
R₁ represents hydrogen, halogen, C₁-C₁₀alkyl, C₁-C₁₀alkoxyl, cyano, COOM or SO₃M,
M being hydrogen or an alkaline or alkaline earth metal, **characterized by** reacting a compound of the formula 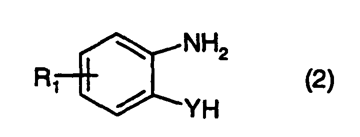
with a dicarboxylic acid of the formula
HOOC-Z-COOH (3)
or an ester thereof, Y, Z and R₁ being as previously defined, in N-methylpyrrolidone or N,N-dimethylacetamide, in the presence of an acidic catalyst and, optionally, in the presence of a secondary solvent capable of removing water from the reaction mixture.

2. A process according to claim 1, in which at least two moles of the compound of formula (2) are reacted with one mole of the dicarboxylic acid of formula (3) or an ester thereof.

3. A process according to claims 1 or 2 for the preparation of a compound of formula (1) in which
Y represents -O-, -S- or -N(R₂)-,
R₂ being hydrogen or C₁-C₄alkyl;
Z is as defined in claim 1 and
R₁ represents hydrogen or C₁-C₄alkyl.

4. A process according to claim 3 in which
Z represents a 2,5-furanyl or a 2,5-thiophenyl residue.

5. A process according to claim 3 in which
Z represents a 4,4'-stilbenyl or a 1,2-ethylenyl residue.

6. A process according to any one of claims 1 to 5 in which reaction of compounds of formulae (2) and (3) is carried out in N-methylpyrrolidone.

7. A process according to any one of claims 1 to 6 in which the acidic catalyst is selected from the group consisting of boric acid, phosphoric acid, titanium C₁-C₄orthoesters or tin derivatives.

8. A process according to claim 7 in which the acidic catalyst is boric acid or a titanium C₁-C₄orthoester.

9. A process according to any one of claims 1 to 8 in which reaction of compounds of the formulae (2) and (3) is carried out within a temperature range of between 100 and 250°C.

10. A process according to claim 6 in which reaction of compounds of the formulae (2) and (3) is carried out within a temperature range of between 150 and 200°C.

11. A process according to any one of claims 1 to 10 in which the secondary solvent capable of removing water from the reaction mixture is selected from the group consisting of toluene, the xylenes and isomeric mixtures thereof and pyridine.

## Patentansprüche

1. Verfahren zur Herstellung einer Verbindung der Formel 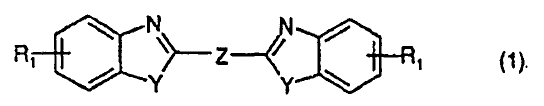 worin
Y -O-, -S- oder -N(R₂)- wiedergibt,
R₂ Wasserstoff, C₁-C₁₀-Alkyl oder Aralkyl darstellt;
Z einen 2,5-Furanyl-, 2,5-Thiophenyl-, 4,4'-Stilbenyl-oder einen 1,2-Ethylenylrest wiedergibt und
R₁ Wasserstoff, Halogen, C₁-C₁₀-Alkyl, C₁-C₁₀-Alkoxyl, Cyano, COOM oder SO₃M wiedergibt,
M Wasserstoff oder ein Alkali- oder Erdalkalimetall darstellt, **gekennzeichnet durch** Umsetzen einer Verbindung der Formel 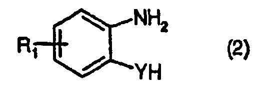
mit einer Dicarbonsäure der Formel
HOOC-Z-COOH (3)
oder einem Ester davon, wobei Y, Z und R₁ wie vorstehend definiert sind, in N-Methylpyrrolidon oder N,N-Dimethylacetamid, in Gegenwart eines sauren Katalysators und gegebenenfalls in Gegenwart eines sekundären Lösungsmittels, das Wasser aus dem Reaktionsgemisch entfernen kann.

2. Verfahren nach Anspruch 1, wobei mindestens 2 Mol der Verbindung der Formel (2) mit einem Mol der Dicarbonsäure der Formel (3) oder einem Ester davon umgesetzt werden.

3. Verfahren nach Ansprüchen 1 oder 2 zur Herstellung einer Verbindung der Formel (1), wobei
Y -O-, -S- oder -N(R₂)- wiedergibt,
R₂ Wasserstoff oder C₁-C₄-Alkyl darstellt;
Z wie in Anspruch 1 definiert ist und
R₁ Wasserstoff oder C₁-C₄-Alkyl wiedergibt.

4. Verfahren nach Anspruch 3, wobei Z einen 2,5-Furanyl- oder einen 2,5-Thiophenylrest wiedergibt.

5. Verfahren nach Anspruch 3, wobei Z einen 4,4'-Stilbenyl- oder einen 1,2-Ethylenylrest wiedergibt.

6. Verfahren nach einem der Ansprüche 1 bis 5, wobei die Reaktion von Verbindungen der Formeln (2) und (3) in N-Methylpyrrolidon ausgeführt wird.

7. Verfahren nach einem der Ansprüche 1 bis 6, wobei der saure Katalysator aus der Gruppe, bestehend aus Borsäure, Phosphorsäure, Titan-C₁-C₄-orthoestern oder Zinnderivaten, ausgewählt ist.

8. Verfahren nach Anspruch 7, wobei der saure Katalysator Borsäure oder ein Titan-C₁-C₄-orthoester ist.

9. Verfahren nach einem der Ansprüche 1 bis 8, wobei die Reaktion von Verbindungen der Formeln (2) und (3) in einem Temperaturbereich zwischen 100 und 250°C ausgeführt wird.

10. Verfahren nach Anspruch 6, wobei die Reaktion von Verbindungen der Formeln (2) und (3) in einem Temperaturbereich zwischen 150 und 200°C ausgeführt wird.

11. Verfahren nach einem der Ansprüche 1 bis 10, wobei das sekundäre Lösungsmittel, das Wasser aus dem Reaktionsgemisch entfernen kann, aus der Gruppe, bestehend aus Toluol, den Xylolen und Isomerengemischen davon und Pyridin, ausgewählt ist.

## Revendications

1. Procédé pour la préparation d'un composé de formule 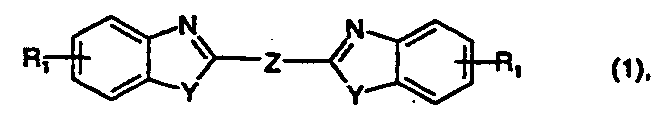 où
Y représente un groupe -O-, -S- ou -N(R₂)-,
R₂ représente un atome d'hydrogène, un groupe alkyle en C₁-C₁₀ ou aralkyle ;
Z représente un résidu 2,5-furanyle, 2,5-thiophényle, 4,4'-stilbényle ou 1,2-éthylényle et
R₁ représente un atome d'hydrogène, un atome d'halogène, un groupe alkyle en C₁-C₁₀, un groupe alkoxy en C₁-C₁₀, cyano, COOM ou SO₃M,
M étant un atome d'hydrogène ou un atome de métal alcalin ou de métal alcalino-terreux, **caractérisé en ce qu'**on fait réagir un composé de formule 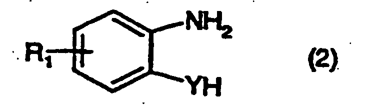
sur un acide dicarboxylique de formule
**HOOC-Z-COOH (3)**
ou un ester de celui-ci, Y, Z et R₁ ont été définis auparavant, dans de la N-méthylpyrrolidone ou du N,N-diméthylacétamide, en présence d'un catalyseur acide et, éventuellement, en présence d'un solvant secondaire capable d'éliminer l'eau à partir du mélange réactionnel.

2. Procédé selon la revendication 1, dans lequel on fait réagir au moins deux moles du composé de formule (2) sur une mole de l'acide dicarboxylique de formule (3) ou un ester de celui-ci.

3. Procédé selon les revendications 1 ou 2 pour la préparation d'un composé de formule (1) dans lequel
Y représente un groupe -O-, -S- ou -N(R₂)-,
R₂ représente un atome d'hydrogène ou un groupe alkyle en C₁-C₄ ;
Z est défini à la revendication 1 et
R₁ représente un atome d'hydrogène ou un groupe alkyle en C₁-C₄.

4. Procédé selon la revendication 3, dans lequel
Z représente un résidu 2,5-furanyle ou 2,5-thiophényle.

5. Procédé selon la revendication 3, dans lequel
Z représente un résidu 4,4'-stilbényle ou 1,2-éthylényle.

6. Procédé selon l'une quelconque des revendications 1 à 5, dans lequel la réaction des composés de formules (2) et (3) est mis en oeuvre dans la N-méthylpyrrolidone.

7. Procédé selon l'une quelconque des revendications 1 à 6, dans lequel le catalyseur acide est pris dans le groupe comprenant l'acide borique, l'acide phosphorique, les orthoesters en C₁-C₄ du titane ou les dérivés de l'étain.

8. Procédé selon la revendication 7, dans lequel le catalyseur acide est l'acide borique ou un orthoester en C₁-C₄ du titane.

9. Procédé selon l'une quelconque des revendications 1 à 8, dans lequel la réaction des composés de formules (2) et (3) est mise en oeuvre dans un domaine de températures compris entre 100 et 250°C.

10. Procédé selon la revendication 6, dans lequel la réaction des composés de formules (2) et (3) est mise en oeuvre dans un domaine de températures compris entre 150 et 200°C.

11. Procédé selon l'une quelconque des revendications 1 à 10, dans lequel le solvant secondaire capable d'éliminer l'eau à partir du mélange réactionnel est pris dans le groupe constitué par le toluène, les xylènes et leurs mélanges d'isomères et la pyridine.
